(19) Europäisches Patentamt

**European Patent Office**

**Office européen des brevets**

(11) Veröffentlichungsnummer: **0 240 590 B1**

(12) **EUROPÄISCHE PATENTSCHRIFT**

(45) Veröffentlichungstag der Patentschrift: **28.08.91**

(21) Anmeldenummer: **86105010.2**

(22) Anmeldetag: **11.04.86**

(51) Int. Cl.⁵: **A61M 25/00**

(54) **Injektions-Absperrventil.**

(43) Veröffentlichungstag der Anmeldung:
**14.10.87 Patentblatt 87/42**

(45) Bekanntmachung des Hinweises auf die
Patenterteilung:
**28.08.91 Patentblatt 91/35**

(84) Benannte Vertragsstaaten:
**AT BE CH DE FR GB IT LI LU NL SE**

(56) Entgegenhaltungen:
**DE-A- 3 142 524        DE-U- 7 812 248**
**US-A- 3 965 910        US-A- 3 996 923**
**US-A- 4 301 811        US-A- 4 506 691**

(73) Patentinhaber: **B. Braun-SSC AG**
**Gerliswilstrasse 74**
**CH-6020 Emmenbrücke(CH)**

(72) Erfinder: **Zaugg, Paul**
**Haldenstrasse 16**
**CH-3432 Lützelflüh(CH)**
Erfinder: **Kiesinger, Werner**
**Hainbuchenweg 10**
**CH-8400 Winterthur(CH)**

(74) Vertreter: **Maspoli, René A.**
**PATENTANWALTSBUREAU R.A. MASPOLI**
**Postfach 191**
**CH-8053 Zürich(CH)**

**Beschreibung**

Die vorliegend beschriebene Erfindung betrifft ein Gleichstrominjektions-Absperrventil zum Einsetzen in eine Infusionsleitung o.ä. Das Ventil weist ein Gehäuse mit Anschlüssen für Ein- und Austritt der genannten Leitung sowie eine Oeffnung zur Einführung einer Injektionsspritze auf. Beim Injizieren mit dem Ventil wird sowohl ein Vermischen der Injektions- mit der Infusionslösung in Richtung Patient vermieden wie auch eine Rückströmung der Injektionslösung zum Infusionsbehälter verunmöglicht.

Injektionsventile zur Verabreichung von Einspritzungen in Infusionsleitungen o.ä. sind bekannt: Beim Injektionsventil gemäss DE Patent 1 216 489 weist der Ventilkörper eine elastische Hülse auf. Der Ventilkörper ist in den Hauptdurchströmungskanal eingesetzt und die Hülse liegt mit ihrem Umfang gegen die Auslassöffnung für Injektionsflüssigkeit abdichtend an. Beim Einpressen von Injektionslösung werden durch den hierbei erzeugten Injektionsdruck die Hülse von der Auslassöffnung im Ventilkörper weggedrückt und das Ventil geöffnet. Das Durchströmen der Infusionsleitung wird durch die Injektion nicht eigentlich beeinflusst.

Bei der genannten Ausführungsform wird zunächst zwischen Kegelansatz der Injektionsspritze und Ventil ein hydrostatischer Druck aufgebaut, mit dem die Ventilkräfte und der gegen das Ventil stehende Systemdruck überwunden werden müssen.

Die bei der Zufuhr von Injektionsflüssigkeit entstehende spezifische Kraft, resultierend aus Reibungskräften in der Injektionsspritze und dem hydrodynamischen Druck zur Offenhaltung des Ventiles, wird in bestimmten Fällen - zum Beispiel bei Verwendung des Injektionsventiles in Venen-Verweilkanülen, die auf der Haut des Patienten fixiert sind - in Form von Flächenpressung auf die Haut des Patienten weitergegeben. Da einem Patienten aus einer Füllung der Injektionsspritze in zeitlichen Abständen auch mehrfach geringe Mengen zugegeben werden, können je nach Grösse der Ventilkräfte erhebliche Mehrfachbelastungen für den Patienten auftreten.

Wird die Zufuhr von Injektionsflüssigkeit beendet, bleibt zunächst der hydrostatische Druck bestehen, der mit den Ventilkräften im Gleichgewicht steht, das heisst, es verbleibt eine Restmenge von Injektionsflüssigkeit zwischen dem Kegelansatz der Injektionsspritze und dem Ventil. Beim Aussetzen der Verabreichung von Injektionsflüssigkeit für längere Zeit - wobei die Injektionsspritze entfernt wird - besteht die Gefahr, dass die restliche Injektionsflüssigkeit kontaminiert und mit der nächsten Injektion dem System und damit dem Patienten zugeführt wird.

Ein weiteres bekanntes Injektionsventil gemäss DE Gebrauchsmuster 78 12 248 enthält einen gummielastischen, geschlitzten Ventilkörper, der in ein Gehäuse spannungsfrei eingesetzt ist. Der Kegelansatz der Injektionsspritze drückt die durch die Schlitzung gebildeten Lappen des Ventilkörpers auseinander, so dass Injektionsflüssigkeit in den Hauptströmungskanal eingeführt werden kann. Auch ist es möglich, Flüssigkeit, zum Beispiel für Analysen, aus dem System über das Ventil zu entnehmen.

Auch bei der Injektion mittels dieses Ventils wird das gleichzeitige Durchströmen der Infusionslösung nicht beeinflusst.

Nach Herausziehen des Kegelansatzes aus dem Ventilkörper schliesst sich die Oeffnung selbsttätig, und es bleibt praktisch keine Restmenge von Injektionsflüssigkeit zwischen Kegelansatz und Ventil zurück. Eine erhöhte Belastung auf der Hautoberfläche des Patienten in Form von Flächenpressung tritt nur einmal beim Einsetzen der Injektionsspritze in die Einstecköffnung auf, während bei weiterer Verabreichung von Injektionsflüssigkeit die Belastung um den erheblichen Anteil der Ventilkräfte reduziert wird. Bei diesem bekannten Injektionsventil ist es jedoch für die die Injektionsspritze handhabende Person nachteilig, dass hohe Kräfte zum Oeffnen des Ventilkörpers mittels des Kegelansatzes der Injektionsspritze erforderlich sind, das heisst, dass es recht schwierig ist, den Kegelansatz der Injektionsspritze in die Einsteck-Öffnung des Injektionsventiles soweit hineinzustecken, bis der Kegelansatz den Ventilkörper mechanisch geöffnet hat. Dies ist darauf zurückzuführen, dass der bekannte Ventilkörper, der spannungsfrei in das Gehäuse eingesetzt ist, eine erhebliche Wandstärke aufweisen muss, damit die Eigenelestizität des Materials ausreicht, um den Schlitz nach Herausziehen des Kegelansatzes zu schliessen bzw. geschlossen zu halten.

Dem Injektionsventil gemäss EP-Anmeldung, Ver. Nr. 0 015 443, liegt die Erfindung zugrunde, den gummielastischen Ventilkörper des Ventiles so auszubilden, dass er bei verhältnismässig dünner Wandstärke und entsprechend erleichterter Oeffnung mittels des Kegelansatzes der Injektionsspritze ausreichende Schliesskraft aufgrund seiner Eigenelastizität hat.

Diese Aufgabe wird im genannten EP-Gesuch dadurch gelöst, dass der Ventilkörper in Form einer elastischen, unter Eigenspannung stehenden Platte ausgebildet ist, die einen an elastischen Bändern aufgehängten Dichtkörper aufweist, und deren Randbereich ein Klemmkörper gegen eine Ringschulter an der Gehäuseauslassöffnung andrückt. Die an ihrem Randbereich festgeklemmt gehaltene Platte kann in ihren elastischen Bändern zum Dichtkörper so dünn ausgebildet sein, dass sie sich mittels des in die Einstecköffnung eingeführten Kegelansatzes der Injektionsspritze leicht und praktisch ohne Kraftaufwand aufdrücken lässt. Der Dichtkörper weicht aufgrund seiner elastischen Aufhängung bzw. Konstruktion bereits bei leichtem Andruck des Kegelansatzes aus und ermöglicht das Zuspritzen von Flüssigkeit. Die Eigenspannung der Platte bringt es mit sich, dass trotz der dünnen Bemessung beim Herausziehen des Kegelansatzes der Injektionsspritze die Oeffnung sich selbsttätig verschliesst und den Hauptdurchströmungskanal flüssigkeitsdicht absperrt. Die Eigenelastizität der eingespannten Platte erlaubt ein beliebig häufiges Zuspritzen von Infusionslösung oder dergleichen bei stets gleichbleibender Dichtwirkung des Ventilkörpers. Dies ist insbesondere bei Venenverweilkanülen mit Zuspritzmöglichkeit wesentlich. Beim Zurückziehen und Entfernen der Injektionsspritze bleibt praktisch keine Restmenge von Injektionsflüssigkeit zwischen dem Kegelansatz der Injektionsspritze und dem Ventil zurück, wodurch die Gefahr einer Zuführung kontaminierter Injektionsflüssigkeit zum Patienten vermieden ist. Das Injektionsventil gestattet ausser einer Zuführung von Flüssigkeit auch eine Flüssigkeitsentnahme aus dem System, zum Beispiel zum Zwecke von Analysen. Die Kombination von Platte und Klemmkörper erlaubt eine Herausnahme der Platte zum Auswechseln gegen eine gleiche neue Platte oder gegen eine Platte mit abgewandeltem Dichtkörper zur Erzielung einer geänderten Durchflussöffnung.

Das DE Patent 1 216 489 lehrt und beansprucht ein Injektionsventil mit einer speziellen Kanülenanordnung. Mit dieser Kanülenanordnung soll eine Injektionsflüssigkeit in eine Kanüle eingeführt werden, die mit einem Behälter für eine Infusionslösung verbunden ist, beispielsweise mit einer Venenkanüle. Die Anordnung soll in die Leitung zwischen der Venenkanüle und der Verbindung zum Behälter mit der Infusionflüssigkeit eingesetzt werden. Die Anordnung hat ein Gehäuse mit einer durchgehenden Leitung für durchströmende Flüssigkeit und eine Seitenöffnung längs der Kanülen-flüssigkeitsleitung, über die eine Injektionsflüssig-keit eintritt. Die Einlassöffnung weist einen konischen Nippel zur Verbindung mit dem Auslasskonus einer Injektionsspritze auf. Ein Körper eines Sperrventils ist aus flexiblem Material und befindet sich im Durchströmungsweg der Kanüle, wobei das Ventil dichtend über die Seiten-Einlassöffnung gelegt ist. Das Ventil wird bei Anwendung eines ausreichend hohen Druckes der Injektionsflüssigkeit von der Seitenöffnung her aus seiner dichtenden Anlage quer über die Oeffnung weggedrückt. Bei einer bevorzugten Ausführungsform ist das Ventil eine mobile manschentenartige Verlängerung ei-nes Rohres, welches das Verbindungteil zum Be-hälter für die Infusionslösung darstellt.

Die Kanülenanordnung gemäss der DE Offenlegungsschrift 2 601 993, deren Erfindungsgegenstand als eine Verbesserung des Injektionsventils gemäss oben besprochenem DE Patent 1 216 486 zu verstehen ist, schlägt eine Kanüle zum Eingeben einer Flüssigkeit aus zwei getrennten Zufuhrleitungen vor, beispielsweise von einem Behälter für eine Infusionslösung und von einer Injektionsspritze aus, und ist so konstruiert, dass Schwierigkeiten vermieden werden. Die Kanüle weist in Kombination auf: ein Gehäuse, eine Leitung für eine Flüssigkeit durch das Gehäuse mit zwei Einlassöffnungen und einen Auslass für eine Flüssigkeit. Eine der Einlassöffnungen ist so geformt, dass sie eine Abgabeeinrichtung zum Zuführen einer Flüssigkeit zum Einlass, und damit zur Durchlassöffnung der Kanüle, aufnehmen kann. Dieser Einlass hat ein Sperrventil, durch das die Strömung nur in einer nach innen gerichteten Richtung durchgelassen wird.

Bei einer bevorzugten Ausführungsform ist der eine Einlass becherförmig geformt, wobei die Seiten- und Stirnwände aus relativ hartem Material, beispielsweise Kunststoff oder Metall, bestehen. Die Einlassöffnung fluchtet mit dem Ende des Bechers, so dass das Becherende jeden Stoss oder Schlageinwirkung der Abgabeeinrichtung (beispielsweise einer Spritzennadel) aufnimmt, wenn diese in den Einlass eingesetzt ist. Die flüssigkeitsführende Verbindung zwischen dem Einlass und der Strömungsleitung für die Flüssigkeit befindet sich in den Seiten des Bechers in Form von einer oder mehreren Oeffnungen durch die Seitenwände. Das oder die Sperrventile schliessen die Oeffnung oder die Oeffnungen dichtend ab. Das Sperrventil besteht aus elastischem Material, beispielsweise elastischem Kunststoff oder Kautschuk, wobei das Material eng anliegend und dichtend die Aussenseite des Bechereinlasses umgibt. Dadurch werden die Seitenöffnungen im Becher abgeschlossen. Das elastische Material kann bei Anwendung eines ausreichenden Flüssigkeitsdrucks vom Bechereinlass sich von den Oeffnungen weg bewegen und diese dadurch öffnen. Dies geschieht beispielsweise, wenn eine Flüssigkeit aus einer Injektionseinrichtung dort injiziert wird.

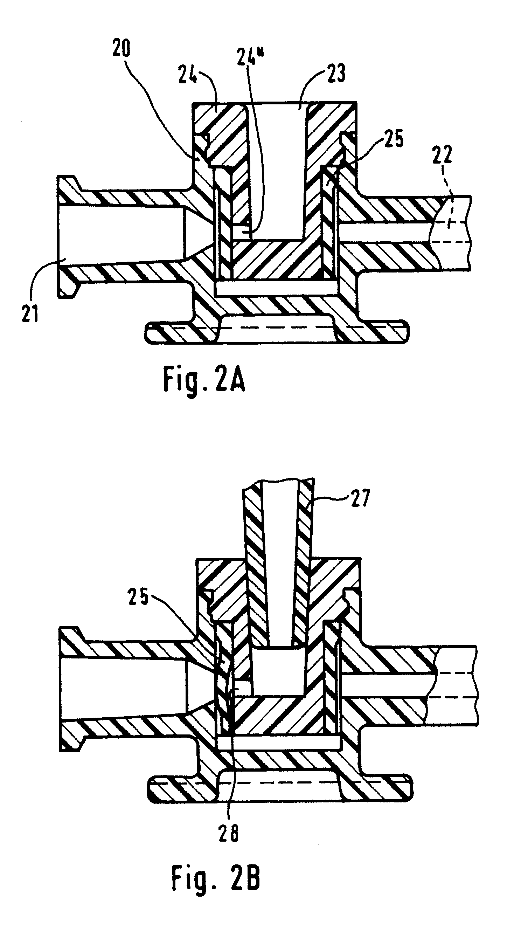

Beiden eben besprochenen Injektionsventilen ist gemeinsam, dass der Rückschlagsicherung tatsächlich ebenfalls keine Beachtung geschenkt wird (Siehe auch Fig. 2 in DE Patent 1 216 489).

Die DE-Al-3 142 524 schliesslich lehrt ein Injektionsinstrument, das immer ein Anschlussstück umfasst, welches offensichtlich dauernd im Blutgefäss eingesteckt bleibt. Das Anschlussstück weist eine innere Abdichtmembrane in Form eines Gummischlauches auf, welche Membrane vor allem die seitlichen Zufuhrstutzen gegen Blutaustritt abdichtet (siehe Seite 5, Zeilen 11 ff).

Gemäss den Figuren 10 und 11 in der hier behandelten DE-Al dichtet die genannte Membrane aber auch den Eingang zum Hauptstutzen ab, wenn z.B. eine Spritze in den Nebenstutzen eingeschoben wird - auch das gleichzeitige Spritzen in Haupt- und Nebenstutzen soll möglich sein. Die Membrane wird bei jeder Einspritzung stark umgestülpt.

Der Stand der Technik betrifft also, wie gezeigt worden ist, hauptsächlich Injektionsventile zur Verabreichung von Einspritzungen in Infusionsleitungen o.ä. Dass bei einer derartigen Injektion noch mindestens eine sehr wesentliche Bedingung erfüllt werden sollte, die Minimalisierung der Veränderungen der Injektionslösung durch Rückmischung mit der Infusionslösung nämlich, ist bislang nicht oder nur völlig ungenügend beachtet worden.

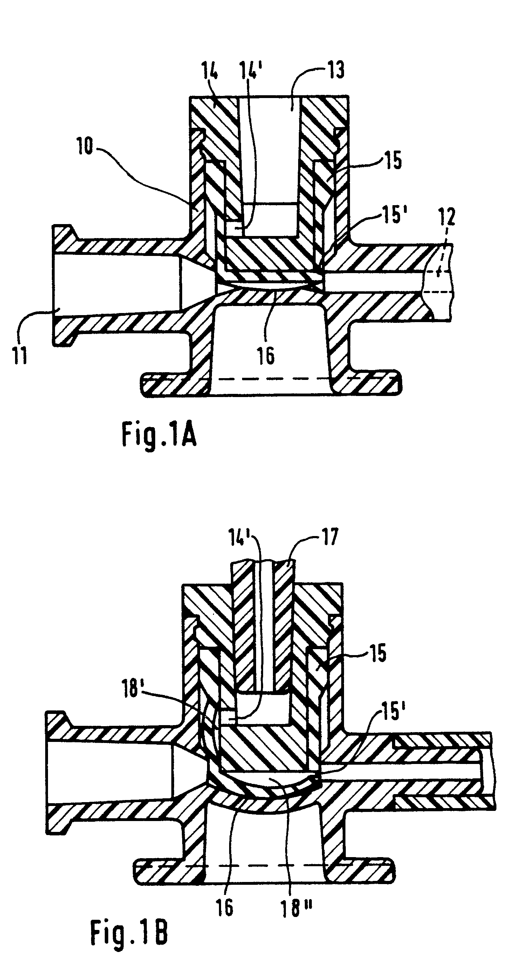

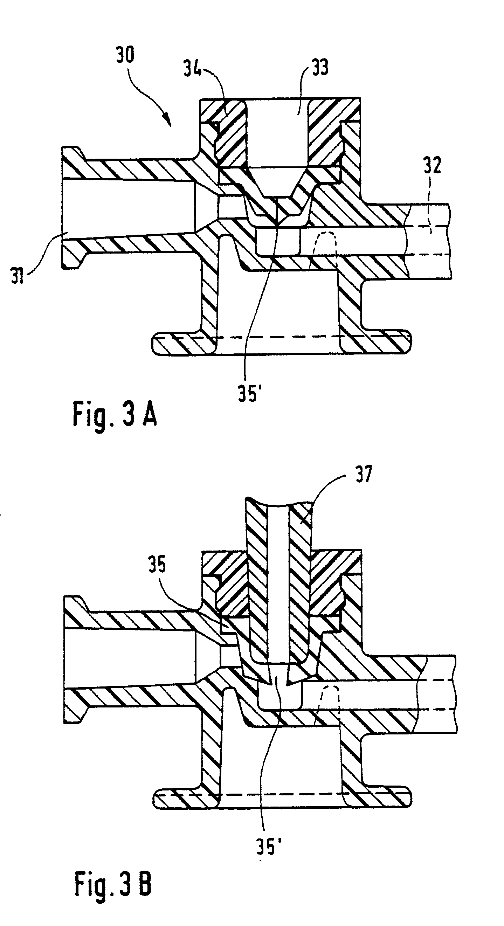

Demgegenüber weist das erfindungsgemässe GleichstrominjektionsAbsperrventil gemäss den Figuren 1 und 3 eine geführte Membrane auf, d.h. eine solche, die in Ruhelage an einem Festkörper anliegt und die in Betriebsstellung sich nur wenig deformiert. Da die Vorrichtung zudem in einer installierten Infusionsleitung o.ä. eingebaut ist, sollten Druckstösse von der Blutseite her keinerlei Schwierigkeiten machen. Dies alles garantiert eine wesentlich erhöhte Betriebssicherheit und die (weiter unten ausführlich dargelegte) zusätzliche Funktion.

Es hat sich nun gezeigt, dass durch die erfindungsgemässe Verbesserung die genannte zusätzliche Funktion ohne Beeinträchtigung der anderen wichtigen Funktionen solcher Vorrichtungen und mit genügender Sicherheit hinsichtlich Betrieb und Keimfreiheit zu garantieren vermag. Die zusätzliche Funktion ist dabei:

- die Rückschlagwirkung gegen die in das Gehäuse eintretende Infusionslösung und somit das Nichtvermischen der Injektions-und der Infusionslösung und
- die Oeffnung der flüssigkeitsführenden Verbindung von der Einspritzkanüle in die Austrittsleitung zum Patienten - d.h. letztlich die gesicherte Verabreichung der Injektion.

Das erfindungsgemässe Gleichstrominjektions-Absperrventil zum Einsetzen in eine Infusionsleitung o.ä., mit einem Gehäuse 10, leitend verbundenen Ein- und Austrittsleitungen 11, 12, Oeffnungskörper 14 mit Einspritzöffnung 13, in welchem Ventil die Einspritzöffnung im wesentlichen senkrecht zur Ebene der beiden genannten Leitungen vorgesehen ist. Ein derartiges Ventil ist aus der DE-A-3142 524 bekannt. Die Erfindung ist weiter dadurch gekennzeichnet, dass der Oeffnungskörper 14 eine setilich angebrachte Durchbohrung 14' aufweist, dass eine Kappenmembran 15 zwischen Gehäuse 10 und Oeffnungskörper 14 so befestigt eingesetzt ist, dass sie den Oeffnungskörper seitlich und unten umgibt, wobei die Kappenmembran am Beginn der Austrittsleitung 12 einen Einschnitt 15' aufweist, und dass zwischen Oeffnungskörper 14 und Gehäuse 10 Ausdehnungsräume für das Kappenmembran 15 vorgesehen sind, wodurch, nach der Einführung einer Injektionskanülenspitze 17 in die Einspritzöffnung 13 und unter der Wirkung des primär hydrodynamischen Injektionsdruckes, die Kappenmembran 15 von der Durchbohrung 14' weggedrückt wird und so über gebildete Räume 18' und 18" zwischen dem Oeffungskörper und der Membran und durch Oeffnen des Einschnittes 15' eine flüssigkeitsführende Verbindung zwischen Injektionskanüle und Austrittsleitung zum Patienten geschaffen wird, wobei gleichzeitig durch Anpressen des Kappenmembrans 15 an eine Sitzfläche 16 im Gehäuse 10 die Eintrittsleitung 11 zum Gehäuse blockiert und der Zustrom von Injektionsflüssigkeit in die genannte Eintrittsleitung verhindert wird.

Eine zweite Ausführungsform des erfindungsgemässen Gleichstrominjektions-Absperrventils zum Einsetzen in eine Infusionsleitung o.ä., mit einem Gehäuse 20, leitend verbundenen Ein- und Austrittsleitungen 21, 22, Oeffnungskörper 24 mit Einspritzöffnung 23, in welchem Ventil die Einspritzöffnung im weseptlichen senkrecht zur Ebene der beiden genannten Leitungen vorgesehen ist, die auch in der DE-A-3142 524 beschrieben ist. Die Erfindung ist weiter dadurch gekennzeichnet, dass der Oeffnungskörper 24 eine seitlich angebrachte Durchbohrung 24" aufweist, dass eine Rundzylindermembran 25 zwischen Gehäuse 20 und Oeffnungskörper 24 so befestigt eingesetzt ist, dass es den Oeffnungskörper seitlich umgibt, und dass zwischen Oeffnungskörper 24 und Gehäuse 20 Ausdehnungsräume für das Rundzylindermembran 25 vorgesehen sind, wodurch, nach der Einführung einer Injektionskanülenspitze 27 in die Einspritzöffnung 23 und unter der Wirkung des primär hydrodynamischen Injektionsdruckes, die Rundzylindermembran 25 in der Gegend der Durchbohrung 24" vom Oeffnungskörper weggedrückt wird, wodurch die Eintrittsleitung 21 geschlossen und, mittels des durch den Druck eröffneten Durchganges 28, eine flüssigkeitsführende Verbindung von der Kanülenspitze nur zur Austrittsleitung geschaffen wird.

Schliesslich ist eine dritte Ausführungsform des erfindungsgemässen Gleichstrominjektions-Absperrventils zum Einsetzen in eine Infusionsleitung, mit einem Gehäuse 30, leitend damit verbundenen Ein- und Austrittsleitungen 31, 32 und Einspritzöffnung 33, in welchem Ventil die Einspritzöffnung im wesentlichen senkrecht zur Ebene der beiden genannten Leitungen vorgesehen ist (siehe DE-A-312 524). Die Erfindung ist weiter dadurch gekennzeichnet, dass in der Einspritzöffnung 33 am Beginn der Austrittsleitung 32 eine Tellermembran 35 mit darin ausgebildetem Schlitz 35' angeordnet ist,

dass zwischen Tellermembran 35 und Gehäuse 30 ein Ausdehnungsraum für die Tellermembran vorgesehen ist, dass nach Einführung der Spitze einer Injektionskanüle in die Einspritzöffnung 33 und unter der Wirkung sowohl des mechanischen Drucks der Kanülenspitze wie auch des hydrodynamischen Drucks der Injektionsflüssigkeit die Tellermembran 35 nach unten und radial gedrückt wird und somit der in ihr befindliche Schlitz 35' geöffnet wird, wobei eine flüssigkeitsführende Verbindung zwischen Injektionskanüle und Austrittsleitung zum Patienten geschaffen wird, wobei gleichzeitig durch Anpressen der Tellermembran an die Oeffnung der Eintrittsleitung 33 im Gehäuse 30 die Eintrittsleitung zum Gehäuse blockiert und folglich der Zustrom von Injektionsflüssigkeit in die genannte Eintrittsleitung verhindert werden

Gemäss den Figuren 1A und 1B weist das Injektions-Absperrventil ein Gehäuse 10, leitend verbundene Ein- und Austrittsleitungen 11, 12 und einen Oeffnungskörper mit Einspritzöffnung 13 auf. Die genannte Oeffnung ist darin im wesentlichen senkrecht zur Ebene der beiden genannten Leitungen vorgesehen, wobei die Ventilvorrichtung durch den als Wand der Injektionsöffnung ausgebildeten Teil des Gehäuses, durch den darin eingesetzten Oeffungskörper 14 mit der darin unten angebrachten, seitlichen Durchbohrung 14' und durch das zwischen den beiden genannten Teilen befestigt eingesetzte Kappenmembran 15 mit Einschnitt 15' gebildet wird. Bei diesem Injektions-Absperrventil wird nun, nach der Einführung der Spitze 17 der Injektionskanüle in die Einspritzöffnung 13 und unter der Wirkung des primär hydrodynamischen Injektionsdruckes, das Membran 15 von der Durchbohrung weggedrückt und so über die Oeffungen 18' und 18" und durch Oeffnen des Einschnittes 15' eine flüssigkeitsleitende Verbindung zwischen Injektionskanüle und Austrittsleitung zum Patienten geschaffen, wobei gleichzeitig durch Anpressen des Membrans 15 an die Sitzfläche 16 im Gehäuse die Eintrittsleitung zum Gehäuse blockiert und der Zustrom von Injektionsflüssigkeit in die genannte Eintrittsleitung verunmöglicht wird.

In der bevorzugten Ausführungsform des erfindungsgemässen Gleichstrominjektions-Absperrventils sind ein Gehäuse 20, leitend verbundene Ein- und Austrittsleitungen 21, 22 und ein Oeffnungskörper mit Einspritzöffnung 23 vorgesehen. Diese Oeffnung liegt im wesentlichen senkrecht zur Ebene der beiden genannten Leitungen, wobei die Ventilvorrichtung durch den als Wand der Injektionsöffnung ausgebildeten Teil des Gehäuses, durch den darin eingesetzten Oeffnungskörper 24 mit der unten und seitlich darin angebrachten Durchbohrung 24' und durch das über den Oeffnungskörper aufgezogene Rundzylindermembran 25 gebildet wird. Bei der Injektion wird, nach der Einführung der Spitze 27 der Injektionskanüle in die Einspritzöffnung 23 und unter der Wirkung des primär hydrodynamischen Injektionsdruckes, das Rundzylindermembran 25 in der Gegend der Durchbohrung 34' vom Oeffungskörper weggedrückt, wodurch die Zuleitung geschlossen und, mittels des durch den Druck eröffneten Durchganges 28, die flüssigkeitsführende Verbindung von der Kanülenspitze nur zur Austrittsleitung geschaffen wird.

Diese Ausführungsform ist in den Figuren 2A und 2B dargestellt.

Schliesslich weist die dritte Ausführungsform des erfindungsgemässen Gleichstrominjektions-Absperrventils zum Einsetzen in eine Infusionsleitung ein Gehäuse 30, leitend damit verbundene Ein-und Austrittsleitungen 31, 32 und eine Einspritzöffnung 33 auf; die Einspritzöffnung ist darin im wesentlichen senkrecht zur Ebene der beiden genannten Leitungen vorgesehen; wobei in der Einspritzöffnung 33 am Beginn der Austrittsleitung 32 eine Tellermembran 35 mit darin ausgebildetem Schlitz 35' so angeordnet ist, dass zwischen Tellermembran 35 und Gehäuse 30 ein Ausdehnungsraum für das Tellermembran vorgesehen ist. Nach Einführung der Spitze einer Injektionskanüle in die Einspritzöffnung 33 wird - unter der Wirkung sowohl des mechanischen Druckes der Kanülenspitze wie auch des hydrodynamischen Druckes der Injektionsflüssigkeit - die Tellermembran 35 nach unten und radial gedrückt und somit der in ihr befindliche Schlitz 35' geöffnet, wobei eine flüssigkeitsführende Verbindung zwischen Injektionskanüle und Austrittsleitung zum Patienten geschaffen wird. Gleichzeitig wird durch Anpressen der Tellermembran an die Oeffnung der Eintrittsleitung 33 im Gehäuse 30 die Eintrittsleitung zum Gehäuse blockiert und folglich der Zustrom von Injektionsflüssigkeit in die genannte Eintrittsleitung verhindert.

Die oben beschriebenen Ausführungsformen des erfindungsgemässen Gleichstrominjektions-Absperrventils zeichnen sich üblicherweise dadurch aus, dass Gehäuse und Oeffnungskörper aus durchsichtigem oder durchscheinendem Kunststoff und das Membran im wesentlichen aus Naturkautschuk bestehen. Sie können mittels üblicher Methoden im wesentlichen keimfrei gemacht werden.

Die hier beschriebenen Gleichstrominjektions-Absperrventile können in Form einer in Infusionsleitungen oder in Ventilen darin einsteckbaren Einheit ausgebildet sein, und gegebenenfalls liegen sie als auf einer Unterlage befestigbare Einheiten vor.

Die Einspritzöffnung der neuen Gleichstrominjektions-Absperrventile ist bevorzugterweise abdeckbar und im wesentlichen leicht zu reinigen.

Verwendung finden die erfindungsgemässen Gleichstrominjektions-Absperrventile vor allem zum Einsetzen in Infusionsleitungen.

**Patentansprüche**

1.  Gleichstrominjektions-Absperrventil zum Einsetzen in eine Infusionsleitung o.ä., mit einem Gehäuse (10), leitend verbundenen Ein- und Austrittsleitungen (11, 12), Oeffnungskörper (14) mit Einspritzöffnung (13), in welchem Ventil die Einspritzöffnung im wesentlichen senkrecht zur Ebene der beiden genannten Leitungen vorgesehen ist, dadurch gekennzeichnet,
    - dass der Oeffnungskörper (14) eine seitlich angebrachte Durchbohrung (14') aufweist,
    - dass eine Kappenmembran (15) zwischen Gehäuse (10) und Oeffnungskörper (14) so befestigt eingesetzt ist, dass sie den Oeffnungskörper seitlich und unten umgibt, wobei die Kappenmembran am Beginn der Austrittsleitung (12) einen Einschnitt (15') aufweist und
    - dass zwischen Oeffnungskörper (14) und Gehäuse (10) Ausdehnungsräume für die Kappenmembran (15) vorgesehen sind,

    wodurch, nach der Einführung einer Injektionskanülenspitze (17) in die Einspritzöffnung (13) und unter der Wirkung des primär hydrodynamischen Injektionsdruckes, die Kappenmembran (15) von der Durchbohrung (14') weggedrückt wird und so über gebildete Räume (18') und (18") zwischen dem Oeffungskörper und der Membran und durch Oeffnen des Einschnittes (15') eine flüssigkeitsführende Verbindung zwischen Injektionskanüle und Austrittsleitung zum Patienten geschaffen wird, wobei gleichzeitig durch Anpressen des Kappenmembrans (15) an eine Sitzfläche (16) im Gehäuse (10) die Eintrittsleitung (11) zum Gehäuse blockiert und der Zustrom von Injektionsflüssigkeit in die genannte Eintrittsleitung verhindert wird.

2.  Gleichstrominjektions-Absperrventil zum Einsetzen in eine Infusionsleitung o.ä., mit einem Gehäuse (20), leitend verbundenen Ein- und Austrittsleitungen (21, 22), Oeffnungskörper (24) mit Einspritzöffnung (23), in welchem Ventil die Einspritzöffnung im wesentlichen senkrecht zur Ebene der beiden genannten Leitungen vorgesehen ist, dadurch gekennzeichnet,
    - dass der Oeffnungskörper (24) eine seitlich angebrachte Durchbohrung (24") aufweist,
    - dass eine Rundzylindermembran (25) zwischen Gehäuse (20) und Oeffnungskörper (24) so befestigt eingesetzt ist, sie dass den Oeffnungskörper seitlich umgibt, und
    - dass zwischen Oeffnungskörper (24) und Gehäuse (20) Ausdehnungsräume für das Rundzylindermembran (25) vorgesehen sind,

    wodurch, nach der Einführung einer Injektionskanülenspitze (27) in die Einspritzöffnung (23) und unter der Wirkung des primär hydrodynamischen Injektionsdruckes, die Rundzylindermembran (25) in der Gegend der Durchbohrung (24") Oeffnungskörper weggedrückt wird, wodurch die Eintrittsleitung (21) geschlossen und, mittels des durch den Druck eröffneten Durchganges (28), eine flüssigkeitsführende Verbindung von der Kanülenspitze nur zur Austrittsleitung geschaffen wird.

3.  Gleichstrominjektions-Absperrventil zum Einsetzen in eine Infusionsleitung, mit einem Gehäuse (30), leitend verbundenen Ein- und Austrittsleitungen (31, 32) und Einspritzöffnung (33), in welchem Ventil die Einspritzöffnung im wesentlichen senkrecht zur Ebene der beiden genannten Leitungen vorgesehen ist, dadurch gekennzeichnet,
    - dass in der Einspritzöffnung (33) am Beginn der Austrittsleitung (32) eine Tellermembran (35) mit darin ausgebildetem Schlitz (35') angeordnet ist,
    - dass zwischen Tellermembran (35) und Gehäuse (30) ein Ausdehnungsraum für die Tellermembran vorgesehen ist,
    - dass nach Einführung der Spitze einer Injektionskanüle in die Einspritzöffnung (33) und unter der Wirkung sowohl des mechanischen Drucks der Kanülenspitze wie auch des hydrodynamischen Drucks der Injektionsflüssigkeit die Tellermembran (35) nach unten und radial gedrückt wird und somit der in ihr befindliche Schlitz (35') geöffnet wird, wobei eine flüssigkeitsführende Verbindung zwischen Injektionskanüle und Austrittsleitung zum Patienten geschaffen wird,

    wobei gleichzeitig durch Anpressen der Tellermembran an die Oeffnung der Eintrittsleitung (33) im Gehäuse (30) die Eintrittsleitung zum Gehäuse blockiert und folglich der Zustrom von Injektionsflüssigkeit in die genannte Eintrittsleitung verhindert werden.

**Claims**

1.  A co-current injection shut-off valve for insertion into a transfusion line or the like, with a housing (10), conductively connected inlet and outlet lines (11, 12), an opening member (14) with an injection aperture (13), in which valve the injection aperture is disposed substantially at right-angles to the plane of the two aforesaid lines, characterised in that

- the opening member (14) comprises a lateral port (14') and in that
- a cap diaphragm (15) is so inserted between the housing (10) and the opening member (14) that it encloses the opening member laterally and at the bottom, the cap diaphragm having at the start of the outlet line (12) a recess (15') and in that
- between the opening member (14) and the housing (10) are expansion spaces for the cap diaphragm (15),

so that after the insertion of an injection cannula tip (17) into the injection aperture (13) and under the action of the primary hydrodynamic injection pressure, the cap diaphragm (15) is forced away from the port (14') and so, via spaces (18') and (18") which are formed between the opening member and the diaphragm and by opening the recess (15') a liquid-conducting connection is created between the injection cannula and the outlet line to the patient, whereby at the same time by pressing the cap diaphragm (15) against a seating surface (16) in the housing (10) the inlet line (11) to the housing is closed off and the inflow of injection fluid into the said inlet line is rendered impossible.

2. A co-current injection shut-off valve for insertion into a transfusion line or the like, with a housing (20), conductively connected inlet and outlet lines (21, 22), an opening member (24) with an injection aperture (23), in which valve the injection aperture is disposed substantially at right-angles to the plane of the two aforesaid lines, characterised in that

- the opening member (24) comprises a lateral port (24') and in that a circular cylinder diaphragm (25) is so inserted between the housing (20) and the opening member (24) that it laterally surrounds the opening member and
- between the opening member (24) and the housing (20) are expansion spaces for the circular cylinder diaphragm (25)

so that after the insertion of an injection cannula tip (27) into the injection aperture (23) and under the action of the primary hydrodynamic injection pressure, the circular cylinder diaphragm (25) is in the region of the port (24") forced away from the opening member so that the inlet line (21) is closed and, by means of the port (28) which is opened up by the pressure, a liquid conveying connection is created from the cannula tip but only to the outlet line.

3. A co-current injection shut-off valve for insertion into a transfusion line, with a housing (30), conductively connected inlet and outlet lines (31, 32) and injection aperture (33), in which valve the injection aperture is disposed substantially at right-angles to the plane of the two aforesaid lines, characterised in that

- in the injection aperture (33) at the start of the outlet line (32) there is a plate-shaped diaphragm (35) with a slot (35') constructed in it,
- and in that between the plate-shaped diaphragm (35) and the housing (30) there is an expansion space for the plate-shaped diaphragm,
- and in that after insertion of the tip of an injection cannula into the injection aperture (33) and under the effect both of the mechanical pressure of the cannula tip and also of the hydrodynamic pressure of the injection fluid the plate-shaped diaphragm (35) is pressed downwardly and radially so that the slot (35') provided in it is opened, a liquid conducting connection being created between the injection cannula and the outlet line to the patient,

so that at the same time by pressing the plate-shaped diaphragm against the opening of the inlet line (33) in the housing (30) the inlet line to the housing is closed off and consequently the inflow of injection fluid into the said inlet line is prevented.

**Revendications**

1. Clapet de fermeture destiné à des injections dans un écoulement continu par insertion dans une conduite de perfusion ou similaire, comprenant un logement (10), des conduits d'entrée et de sortie (11, 12) reliés de façon à conduire les liquides et un corps d'ouverture (14) muni d'une ouverture d'injection (13), clapet dans lequel l'ouverture d'injection est prévue pour l'essentiel perpendiculairement au plan des deux conduits précités, caractérisé par le fait :

- que le corps d'ouverture (14) présente un perçage traversant (14') ménagé latéralement,
- qu'une membrane en capuchon (15) est insérée et fixée entre le logement (10) et le corps d'ouverture (14) de telle manière qu'elle entoure le corps d'ouverture latéralement et vers le bas, la membrane en capuchon présentant une entaille (15') au début du conduit de sortie (12), et :
- que des volumes d'expansion sont prévus pour la membrane en capuchon (15) entre le corps d'ouverture (14) et le logement (10),

grâce à quoi, après l'introduction d'une pointe de canule à injection (17) dans l'ouverture d'injection (13), et sous l'effet de la pression d'injection hydrodynamique primaire, la membrane en capuchon (15) est repoussée du perçage traversant (14'), et il se crée, par l'intermédiaire de chambres (18' et 18") ainsi formées entre le corps d'ouverture et la membrane, et grâce à l'ouverture de l'entaille (15'), une liaison conduisant le liquide entre la canule d'injection et le conduit de sortie vers le patient, cependant que, simultanément, grâce au fait que la membrane en capuchon (15) est appuyée contre une surface de siège (16) ménagée dans le logement (10), le conduit d'entrée (11) vers le logement est bloqué et l'amenée de liquide d'injection dans le conduit d'entrée précité est empêchée.

2. Clapet de fermeture destiné à des injections dans un écoulement continu par insertion dans une conduite de perfusion ou similaire, comprenant un logement (20), des conduits d'entrée et de sortie (21, 22) reliés de façon à conduire les liquides et un corps d'ouverture (24) muni d'une ouverture d'injection (23), clapet dans lequel l'ouverture d'injection est prévue pour l'essentiel perpendiculairement au plan des deux conduits précités, caractérisé par le fait :

- que le corps d'ouverture (24) présente un perçage traversant (24") ménagé latéralement,
- qu'une membrane en cylindre de révolution (25) est insérée et fixée entre le logement (20) et le corps d'ouverture (24) de telle manière qu'elle entoure latéralement le corps d'ouverture, et :
- que des volumes d'expansion sont prévus pour la membrane en cylindre de révolution (25) entre le corps d'ouverture (24) et le logement (20),

grâce à quoi, après l'introduction d'une pointe de canule à injection (27) dans l'ouverture d'injection (23), et sous l'effet de la pression d'injection hydrodynamique primaire, la membrane en cylindre de révolution (25) est repoussée du corps d'ouverture dans la région du perçage traversant (24"), grâce à quoi le conduit d'entrée (21) est fermé et, au moyen du passage (28) ouvert par la pression, une liaison conduisant le liquide est créée depuis la pointe de canule d'injection et uniquement vers le conduit de sortie.

3. Clapet de fermeture destiné à des injections dans un écoulement continu par insertion dans une conduite de perfusion ou similaire, comprenant un logement (30), des conduits d'entrée et de sortie (31, 32) reliés de façon à conduire les liquides et une ouverture d'injection (33), clapet dans lequel l'ouverture d'injection est prévue pour l'essentiel perpendiculairement au plan des deux conduits précités, caractérisé par le fait :

- qu'une membrane en cuvette (35) dans laquelle est formée une fente (35') est disposée dans l'ouverture d'injection (33) au début du conduit de sortie (32),
- qu'un volume d'expansion est prévu pour la membrane en cuvette entre la membrane en cuvette (35) et le logement (30),
- qu'après l'introduction de la pointe d'une canule à injection dans l'ouverture d'injection (33), et sous l'effet, tant de la pression mécanique de la pointe de la canule que de la pression d'injection hydrodynamique primaire, la membrane en cuvette (35) est poussée vers le bas et radialement et la fente (35') ménagée dans celle-ci est ainsi ouverte, cependant qu'une liaison conduisant le liquide est créée entre la canule d'injection et le conduit de sortie vers le patient,

et cependant que, simultanément, grâce au fait que la membrane en cuvette est appuyée contre l'ouverture du conduit d'entrée (31) ménagée dans le logement (30), le conduit d'entrée est bloqué vers le logement et, en conséquence, l'amenée de liquide d'injection dans le conduit d'entrée précité est empêchée.

Fig.1A

Fig.1B

Fig. 2A

Fig. 2B

Fig. 3 A

Fig. 3 B